# DEMANDE DE BREVET EUROPEEN

(11) **EP 4 245 441 A1**
(43) Date de publication de la demande: **20.09.2023**
(21) Numéro de dépôt: 23162752.2
(22) Date de dépôt: 17.03.2023
(51) Int. Cl.: B22F 10/28, A61L 27/06, B33Y 10/00, B33Y 70/00, B33Y 80/00, C22C 1/04, C22C 14/00, C22F 1/18, A61L 31/02

(54) **PROCÉDÉ DE FABRICATION D'UN ALLIAGE BIOCOMPATIBLE PAR FUSION LASER SUR LIT DE POUDRE ET DISPOSITIFS MÉDICAUX RÉALISÉS À PARTIR DE CET ALLIAGE**

(30) Priorité: 17.03.2022 FR 2202376
(71) Demandeur: SLS France, 35136 Saint-Jacques-de-la-Lande (FR)
(72) Inventeur: SCHAAL, HUGO, 35200 RENNES (FR); GLORIANT, THIERRY, 35510 CESSON SEVIGNE (FR); DUBOIS, AXEL, 35650 LE RHEU (FR)
(74) Mandataire: Ermeneux, Bertrand

(57) **Abrégé**

L'invention concerne un procédé de fabrication additive d'une pièce en alliage quaternaire Ti-Zr-Nb-Sn biocompatible par fusion sur lit de poudre et un dispositif médical formé à partir de cette pièce.

Selon l'invention, un tel procédé comprend les étapes de :
- mélange de particules de poudres de titane, de zirconium, de niobium et d'étain de tailles micrométriques dans des proportions respectives de 60,5 à 78,5 %, de 15 à 25 %, de 5 à 12 % et de 1,5 à 2,5 % exprimées en pourcentage atomique ;
- fusion d'un lit dudit mélange de particules de poudres par un faisceau laser ou par un faisceau d'électron de sorte à obtenir ladite pièce.

## Description

### 1. Domaine de l'invention

Le domaine de l'invention est celui de la fabrication additive métallique par fusion laser.

Plus précisément, l'invention concerne un procédé de fabrication additive d'une pièce en alliage quaternaire Ti-Zr-Nb-Sn par fusion sur lit de poudre métallique. L'invention trouve notamment une application en chirurgie de reconstruction maxillo-faciale et en implantologie dentaire ou orthopédique.

### 2. Etat de la technique

Les alliages semi-précieux ou non-précieux de Nickel-chrome et de Cobalt-chrome sont couramment utilisés pour l'élaboration des implants dentaires.

Le nickel présente cependant l'inconvénient d'être allergisant pour l'organisme. Par ailleurs, le cobalt a été classé récemment CMR, ou en d'autres termes est considéré comme une substance cancérigène, mutagène et toxique pour la reproduction, par le comité d'évaluation des risques de l'Agence européenne des produits chimiques.

Il s'avère donc nécessaire de trouver des alternatives biocompatibles aux alliages Nickel-chrome et Cobalt-chrome.

Depuis 1970, on développe des alliages à base de titane pour l'implantologie dentaire et orthopédique.

Un inconvénient des alliages à base de titane connus est que la rigidité des implants élaborés avec ces alliages est largement supérieure à celle des tissus osseux. Ceci provoque des contraintes à l'interface entre l'implant et les tissus osseux par un phénomène de « stress-shielding », qui a pour conséquence une diminution de la densité du cortex du tissu osseux et une résorption de ceux-ci et par conséquence un affaiblissement de la liaison entre les tissus osseux et l'implant, ce qui affecte la stabilité de l'implant et entraine à long terme un échec du traitement implantaire.

On a notamment proposé des alliages de titane et de niobium pouvant incorporer en outre du zirconium et/ou de l'étain, tels que les alliages Ti-Nb, TNZ(Ti-Nb-Zr), TNZT (Ti-Nb-Zr-Ta) et Ti2448 (Ti-24Nb-4Zr-8Sn). Ces alliages, du type β-métastable, présentent l'avantage d'être constitués d'éléments au caractère biocompatible et de posséder un bas module d'élasticité. D'autre part, certaines compositions sont connues pour présenter une transformation martensitique induite sous contrainte qui peut être avantageusement mise à profit pour améliorer encore les propriétés de ces alliages, notamment pour les dispositifs médicaux.

Un inconvénient de ces alliages à base de titane et de niobium connus est qu'il faut porter le niobium à une température très élevée (d'environ 2477°C) pour le faire fondre. Ainsi des infondus solides peuvent subsister au sein de la matrice métallique, notamment si on envisage de les élaborer directement par fabrication additive.

Dans le cadre de la fabrication additive de dispositifs médicaux, des comportements superélastique ou à mémoire de forme, et des effets TRIP et/ou TWIP ont été rapportés dans de rares cas pour des nuances de titane β. Récemment, un procédé de fabrication additive « in situ », consistant en une fusion directe d'un mélange de poudres élémentaires, a été proposé.

Un inconvénient de cette technique de fabrication additive connue, qui offre une grande flexibilité dans la fabrication des pièces, est la présence d'inclusions d'éléments à haut point de fusion dans la matrice métallique. Celles-ci entraînent des inhomogénéités chimiques au sein de l'alliage, ce qui fait varier localement sa composition chimique, et par conséquent modifie le caractère métastable de celui-ci, comme décrit par M. Fischer, « Élaboration in situ d'alliages de titane et de structures architecturées par fabrication additive : application aux dispositifs médicaux implantables ». Université de Lorraine; École doctorale C2MP - Chimie mécanique matériaux physique (Lorraine), 2017.

### 3. Objectifs de l'invention

L'invention a donc notamment pour objectif de pallier les inconvénients de l'art antérieur cités ci-dessus.

Plus précisément l'invention a pour objectif de fournir une technique de fabrication par technologie additive de pièces en alliage à base de titane biocompatible du type β-métastable sans présence d'infondu, qui ne soit pas nocif pour le corps humain.

Un objectif de l'invention est également de fournir une telle technique qui permette de fabriquer des pièces sur mesure.

Dans au moins un mode de réalisation particulier de l'invention, un objectif de l'invention est de fournir une technique de fabrication d'un alliage qui présente une transformation martensitique sous contrainte de la phase cubique centrée β vers la phase orthorhombique α", permettant de lui conférer les propriétés suivantes : super-élasticité, mémoire de forme et/ou des propriétés d'un alliage à grandes déformations combinant des effets TRIP (acronyme de « Transformation Induced plasticity » en anglais) et TWIP (acronyme de « Twinning Induced plasticity » en anglais).

Un autre objectif de l'invention est de fournir une telle technique qui permette d'obtenir un alliage présentant un module d'élasticité inférieur ou égal à 50 GPa et s'approchant du module d'élasticité des tissus biologiques (entre 10 et 30Gpa).

Un autre objectif de l'invention est de fournir une telle technique qui soit simple à mettre en oeuvre et d'un coût de revient réduit.

### 4. Exposé de l'invention

Ces objectifs, ainsi que d'autres qui apparaitront par la suite sont atteints à l'aide d'un procédé de fabrication additive d'une pièce en alliage quaternaire Ti-Zr-Nb-Sn par fusion sur lit de poudre métallique comprenant les étapes de :
- mélange de particules de poudres de titane, de zirconium, de niobium et d'étain de tailles micrométriques dans des proportions respectives de 60,5 à 78,5 %, de 15 à 25 %, de 5 à 12 % et de 1,5 à 2,5 % exprimées en pourcentage atomique ;
- fusion d'un lit dudit mélange de particules de poudres par un faisceau laser de sorte à obtenir ladite pièce.

Ainsi, l'invention propose une technique de fabrication de pièces sur mesure, grâce à la technique de fusion laser sur lit de poudre métallique, en un nouvel alliage à base de titane biocompatible, sans danger pour le corps humain, β-métastable sans infondu et à bas module d'élasticité, pouvant en outre être super-élastique et présenter une haute ductilité.

Il convient de noter que le procédé selon l'invention permet par ailleurs avantageusement de mettre en oeuvre des poudres élémentaires de métaux commercialement disponibles présentant une grande pureté, sans devoir mettre en oeuvre une poudre préalablement pré-alliée obtenue par une technique d'atomisation complexe et coûteuse. On notera en outre que la mise en oeuvre d'une quantité de niobium plus faible que dans les compositions connues, et sa substitution par du zirconium, élément ayant un effet stabilisant sur la phase β du titane, permet de réduire les quantités d'inclusions métalliques et d'assurer une homogénéité chimique macroscopique.

La technique proposée par l'invention s'avère ainsi particulièrement adaptée pour la fabrication de prothèses et d'implants dentaires, de dispositifs orthodontiques, de prothèses et d'implants orthopédiques pour le genou, l'épaule, la cheville ou la hanche, d'implants pour la colonne vertébrale, de plaques d'ostéosynthèse ou d'implants pour la chirurgie maxillo-faciale, ou de plaques d'ostéosynthèse pour l'orthopédie du membre inférieur. En outre, le procédé selon l'invention permet la fabrication de dispositifs médicaux poreux sans structure lattice, de façon à faciliter l'infiltration cellulaire et osseuse.

De préférence, lors de ladite étape de mélange, on mélange 65 à 71% en pourcentage atomique de particules de poudre de titane, 21,5 à 22,5 %, et de préférence 22%, en pourcentage atomique de particules de poudre de zirconium, 5 à 11% en pourcentage atomique de particules de poudre de niobium et 1,5 à 2,5 %, et de préférence 2%, en pourcentage atomique de particules de poudre d'étain.

Dans un mode de réalisation avantageux de l'invention, au moins 99,9% desdites particules de titane, de zirconium, de niobium ont une taille respectivement comprise entre 15 et 45 micromètres, entre 10 et 45 micromètres et entre 10 et 63 micromètres et au moins 99,7% desdites particules d'étain sont de tailles inférieures à 45 micromètres.

Dans un mode de réalisation avantageux de l'invention, lesdites particules de titane, de zirconium et de niobium ont une taille médiane comprise respectivement entre 35,2 µm et 36,2µm, entre 31,5µm et 32,5µm et entre 29,8 et 30,0µm.

Selon un aspect particulier de l'invention, lors ladite étape de fusion d'un lit dudit mélange, la puissance du faisceau laser est comprise entre 200 et 250 W, la vitesse de balayage dudit faisceau laser est comprise entre 300 et 380 mm.s⁻¹, la distance entre deux passes successives est comprise entre 58 et 62 µm et l'épaisseur de la couche de particules de poudre fusionnées est comprise entre 15 et 30 µm.

Dans un mode de réalisation avantageux de l'invention, la puissance du faisceau laser est sensiblement égale à 200 W, la vitesse de balayage dudit faisceau laser est sensiblement égale à 303 mm.s⁻¹, la distance entre deux passes successives est sensiblement égale à 60 µm et l'épaisseur de la couche de particules de poudre fusionnées est sensiblement égale à 20 µm.

De façon avantageuse, un procédé tel que décrit ci-dessus comprend en outre une étape de traitement thermique sous vide secondaire destinée à conférer à ladite pièce un comportement à mémoire de forme, un comportement super-élastique ou un effet TRIP et/ou TWIP comprenant une étape de chauffage de ladite pièce à une température prédéterminée comprise entre 700 et 900°C suivie d'une étape de trempe de la pièce chauffée dans de l'eau à température ambiante.

Dans un mode de réalisation particulier de l'invention, lors de ladite étape de chauffe la température de la pièce est augmentée de 8 à 12°C, et de préférence de 10°C par minute jusqu'à atteindre ladite température prédéterminée et en ce que ladite pièce est maintenu pendant sensiblement 30 minutes à ladite température prédéterminée avant ladite étape de trempe.

Dans un mode de réalisation avantageux de l'invention, ladite étape de fusion sur lit de poudre est une étape de fusion sélective sur lit de poudre. L'invention concerne également un dispositif médical, tel qu'une prothèse dentaire ou orthopédique, un implant dentaire, un implant orthopédique, un implant pour la colonne vertébrale ou une plaque d'ostéosynthèse, formé à partir d'une pièce en alliage quaternaire Ti-Zr-Nb-Sn obtenue par un procédé de fabrication tel que décrit ci-dessus.

### 5. Liste des figures

D'autres caractéristiques et avantages de l'invention apparaitront plus clairement à la lecture de la description suivante d'un mode de réalisation de l'invention, donné à titre de simple exemple illustratif et non limitatif, et des dessins annexés parmi lesquels :
- la figure 1 est une représentation synoptique, sous forme de diagramme-bloc, des étapes d'un exemple de procédé de fabrication d'une plaque d'ostéosynthèse selon l'invention ;
- la figure 2 est une courbe de traction cyclée d'un alliage obtenu par le procédé de fabrication présenté en référence à la figure 1 ;

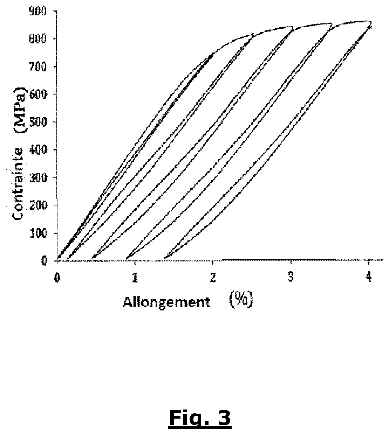

- la figure 3 est une courbe de traction cyclée d'un alliage obtenu par une variante du procédé de fabrication présenté en référence à la figure 1, dans lequel lors de l'étape de traitement thermique post-fabrication, l'alliage a été porté et maintenu à une température de 900°C avant d'être trempé ;
- la figure 4 est une cartographie d'orientation des grains de la microstructure de l'alliage obtenu par le procédé de fabrication présenté en référence à la figure 1.

### 6. Description détaillée de l'invention

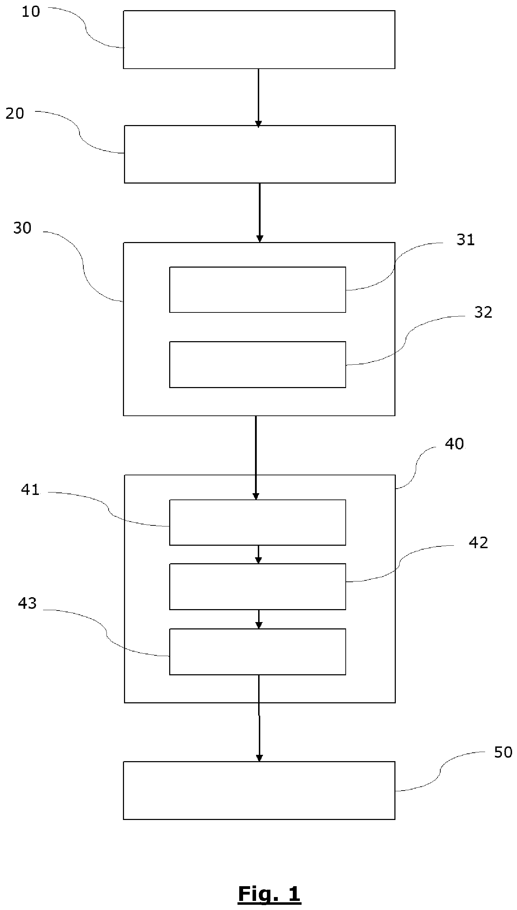

On a illustré sur la figure 1 les étapes d'un exemple de procédé de fabrication d'une plaque d'ostéosynthèse en alliage quaternaire Ti-Zr-Nb-Sn selon l'invention, sous forme de diagramme bloc.

Dans une étape 10, on procède à une pesée :
i) d'une première quantité prédéterminée d'une poudre de titane Ti composée à plus de 99,7% de particules sensiblement sphériques présentant une distribution de tailles de particules comprise entre 15 et 45 mm et un diamètre médian D50 égal à 35,7µm,
ii) d'une deuxième quantité prédéterminée d'une poudre de zirconium Zr composée à plus de 99,7% de particules sensiblement sphériques présentant une distribution de tailles de particules comprise entre 10 et 45 mm et un diamètre médian D50 égal à 32µm,
iii) d'une troisième quantité prédéterminée d'une poudre de niobium Nb composée à plus de 99,9% de particules sensiblement sphériques présentant une distribution de tailles de particules comprise entre 10 et 63 mm et un diamètre médian D50 égal à 29,9µm, et
iv) d'une quatrième quantité prédéterminée d'une poudre d'étain composée à plus de 99,9% de particules présentant une taille inférieure à 45 mm.

Les valeurs respectives de ces première, deuxième, troisième et quatrième quantités déterminées de poudres ont été calculées de sorte à pouvoir obtenir un mélange contenant du titane, du zirconium, du niobium et de l'étain dans des proportions respectives, exprimées en pourcentage atomique, de 67 %, 22 %, 9 % et 2 %. On a par ailleurs ajusté ces première, deuxième, troisième et quatrième quantités déterminées de poudre de titane, de zirconium, de niobium et d'étain de sorte que leur masse cumulée soit égale à 2kg, dans ce mode de réalisation de l'invention.

Dans d'autres modes de réalisation avantageux de l'invention, il peut également être envisagé que les proportions respectives, exprimées en pourcentage atomique, de titane, de zirconium, de niobium et d'étain soient de 65%, 22%, 11% et 2% ou de 69%, 22%, 7%, 2% ou encore de 71%, 22%, 5% et 2%, par exemple.

Les critères de choix qui ont conduit à sélectionner ces poudres sont notamment les suivants :
- les particules de zirconium ont été choisies d'une taille légèrement inférieure à celle des particules de titane, compte-tenu du fait que la température de fusion du zirconium est légèrement supérieure à celle du titane ;
- Compte-tenu de la température élevée de fusion du niobium, on a sélectionné de préférence des particules de niobium fines pour faciliter leur fusion ;
- par souci d'homogénéité structurelle, les particules d'étain ont été choisies de tailles proches de celles des autres poudres.

On mélange ensuite dans une étape 20 les quantités prédéterminées de poudres de titane, de zirconium, de niobium et d'étain dans un mélangeur Turbula T2F a une vitesse de 34 tours par minute, pendant deux heures, afin d'obtenir un mélange de 2kg de poudres d'une homogénéité convenable.

On verse ensuite ce mélange dans une machine de fusion laser sélective sur lit de poudre SLM 125(marque déposée), mettant en oeuvre une technique de fusion SLM (acronyme de « Selective Laser Melting» en anglais) et présentant un dispositif de chauffe de la plaque support du lit de poudre, dans laquelle ce mélange de poudres va être fondu par couches par l'énergie du faisceau d'un laser (étape 30).

Dans cette machine, on a chargé un modèle 3D de la plaque d'ostéosynthèse à obtenir lors d'une étape 31. Ce modèle 3D a été élaboré à partir d'une image de la zone de fracture à réduire obtenue à l'aide d'un scanner 3D à partir duquel une simulation de la pose de la plaque d'ostéosynthèse a été testée. Ceci permet d'obtenir un modèle 3D de plaque d'ostéosynthèse au plus proche de la morphologie du patient.

Par ailleurs, on a réglé lors d'une étape 32, les paramètres du laser à haute puissance de cette machine, destiné à venir balayer les couches successives du lit de poudre, aux valeurs suivantes :
- P = 200 W
- V= 303,03 mm.s⁻¹
- H= 60 µm
- E= 20 µm
- Ed= 550 J/mm³
où P est la puissance du laser, V est la vitesse de balayage du laser, H est la distance entre deux passes lasers successives, E est l'épaisseur de la couche de poudre fusionnée, et E_{d} représente l'énergie volumique délivrée au cours de la fusion.

Il convient de noter que ces paramètres permettent d'obtenir une plaque d'ostéosynthèse dont la densité est supérieure à 99,3% et présentant moins de 0,04% d'infondus en volume et dont la matrice métallique présente une homogénéité chimique convenable.

Une fois les différentes couches fondues à l'aide du faisceau laser pour former une plaque d'ostéosynthèse massive, la poudre excédentaire est retirée. Dans une étape 40, on procède à un traitement thermique de la plaque d'ostéosynthèse encore fixé sur la plaque support.

Ce traitement thermique consiste en un traitement dit de trempe sous vide secondaire à 10⁻⁸ mbar, comprenant une étape de chauffe contrôlée (étape 41) dans laquelle la plaque d'ostéosynthèse est montée en température de 10°C/mn jusqu'à atteindre une température de 700°C, puis est maintenue à cette température pendant 30 minutes (étape 42), et enfin est refroidie rapidement jusqu'à la température ambiante en la trempant dans de l'eau à température ambiante (étape 43). Ce traitement thermique permet de figer la phase β à température ambiante.

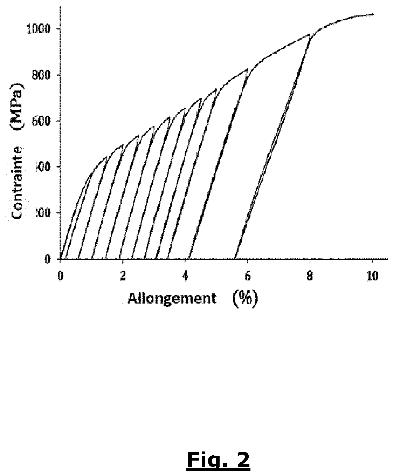

L'alliage ainsi obtenu après trempe présente un effet TRIP responsable d'une consolidation très importante. Son taux d'écrouissage maximum est par ailleurs d'environ 13 GPa et son module d'élasticité est de 44 GPa, comme l'ont montré les résultats d'essais à la traction d'une éprouvette formée de cet alliage, représentés sur la figure 2.

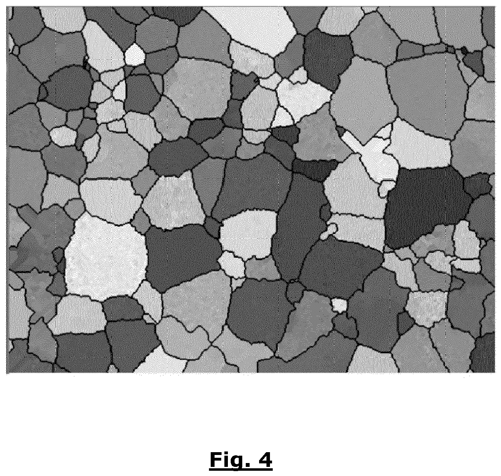

Une cartographie de l'orientation des grains obtenue par une technique de diffraction d'électrons rétrodiffusés (aussi connu par son acronyme EDSB, pour « Electron Back Scattered Diffraction » en anglais) a par ailleurs été réalisée pour cet alliage et est illustrée sur la figure 4.

Dans une variante de ce mode de réalisation particulier de l'invention, la plaque d'ostéosynthèse peut être chauffée jusqu'à 900°C, afin de lui conférer un comportement super-élastique comme l'illustre la figure 4, qui est une courbe de traction cyclée obtenue lors d'un essai à la traction d'une éprouvette formée du même alliage auquel on a fait subir un traitement par trempe en le portant préalablement à 900°C pendant 30 minutes, à partir de laquelle on a pu déterminer que cet alliage après traitement thermique présente une déformation recouvrable d'environ 2,6% et un module d'élasticité de 41 GPa. Dans encore une variante de ce mode de réalisation particulier de l'invention, il peut également être prévu d'effectuer une reprise d'usinage sur une partie de la surface de la plaque d'ostéosynthèse, par exemple afin d'améliorer son état de surface.

Dans une étape 50, la plaque d'ostéosynthèse est détachée de la plaque support par découpe, usinage ou par électroérosion à fil et est désormais prête à l'emploi.

## Revendications

1. Procédé de fabrication additive d'une pièce en alliage quaternaire Ti-Zr-Nb-Sn par fusion sur lit de poudre métallique comprenant les étapes de :
- mélange de particules de poudres de titane, de zirconium, de niobium et d'étain de tailles micrométriques dans des proportions respectives de 60,5 à 78,5 %, de 15 à 25 %, de 5 à 12 % et de 1,5 à 2,5 % exprimées en pourcentage atomique ;
- fusion d'un lit dudit mélange de particules de poudres par un faisceau laser de sorte à obtenir ladite pièce.

2. Procédé de fabrication selon la revendication 1, caractérisé en ce lors de ladite étape de mélange, on mélange 65 à 71% en pourcentage atomique de particules de poudre de titane, 21,5 à 22,5 %, et de préférence 22%, en pourcentage atomique de particules de poudre de zirconium, 5 à 11% en pourcentage atomique de particules de poudre de niobium et 1,5 à 2,5 %, et de préférence 2%, en pourcentage atomique de particules de poudre d'étain.

3. Procédé de fabrication selon l'une quelconque des revendications 1 et 2, **caractérisé en ce que** au moins 99,9% desdites particules de titane, de zirconium et de niobium ont une taille respectivement comprise entre 15 et 45 micromètres, entre 10 et 45 micromètres et entre 10 et 63 micromètres et au moins 99,7% desdites particules d'étain sont de taille inférieure à 45 micromètres.

4. Procédé de fabrication selon l'une quelconque des revendications 1 à 3, **caractérisé en ce qu'**au moins 99,9% desdites particules de titane, de zirconium et de niobium ont une taille médiane comprise respectivement entre 35,2 µm et 36,2µm, entre 31,5µm et 32,5µm et entre 29,8 et 30,0µm.

5. Procédé de fabrication selon l'une quelconque des revendications 1 à 4, **caractérisé en ce que**, pour ladite étape de fusion d'un lit dudit mélange, la puissance du faisceau laser est comprise entre 200 et 250 W, la vitesse de balayage dudit faisceau laser est comprise entre 300 et 380 mm.s⁻¹, la distance entre deux passes successives est comprise entre 58 et 62 µm et l'épaisseur de la couche de particules de poudre fusionnées est comprise entre 15 et 30 µm.

6. Procédé de fabrication selon la revendication 5, **caractérisé en ce que** la puissance du faisceau laser est sensiblement égale à 200 W, la vitesse de balayage dudit faisceau laser est sensiblement égale à 303 mm.s⁻¹, la distance entre deux passes successives est sensiblement égale à 60 µm et l'épaisseur de la couche de particules de poudre fusionnées est sensiblement égale à 20 µm.

7. Procédé de fabrication selon l'une quelconque des revendications 1 à 6, **caractérisé en ce qu'**il comprend en outre une étape de traitement thermique sous vide secondaire destinée à conférer à ladite pièce un comportement à mémoire de forme, un comportement super-élastique ou un effet TRIP et/ou TWIP comprenant une étape de chauffage de ladite pièce à une température prédéterminée comprise entre 700 et 900°C suivie d'une étape de trempe de la pièce chauffée dans de l'eau à température ambiante.

8. Procédé de fabrication selon la revendication 7, **caractérisé en ce que** lors de ladite étape de chauffe la température de la pièce est augmentée de 8 à 12°C, et de préférence de 10°C par minute jusqu'à atteindre ladite température prédéterminée et **en ce que** ladite pièce est maintenu pendant sensiblement 30 minutes à ladite température prédéterminée avant ladite étape de trempe.

9. Procédé de fabrication selon l'une quelconque des revendications 1 à 8, **caractérisé en ce que** ladite étape de fusion sur lit de poudre est une étape de fusion sélective sur lit de poudre.

10. Dispositif médical, tel qu'une prothèse dentaire ou orthopédique, un implant dentaire, un implant orthopédique, un implant pour la colonne vertébrale ou une plaque d'ostéosynthèse, formé à partir d'une pièce en alliage quaternaire Ti-Zr-Nb-Sn obtenue par un procédé de fabrication selon l'une quelconque des revendications 1 à 9.
